# EUROPÄISCHE PATENTSCHRIFT

(11) **EP 0 716 072 B1**
(45) Veröffentlichungstag und Bekanntmachung des Hinweises auf die Patenterteilung: **07.01.1998**
(21) Anmeldenummer: 95116082.9
(22) Anmeldetag: 12.10.1995
(51) Int. Cl.: C07C 59/125, C07C 59/68

(54) **Konzentrierte fliessfähige Polyethercarboxylate**
Concentrated free-flowing polyether carboxylates
Polyéthercarboxylates concentrés à écoulement libre

(30) Priorität: 08.12.1994 DE 4443664
(43) Veröffentlichungstag der Anmeldung: 12.06.1996
(73) Patentinhaber: HÜLS AKTIENGESELLSCHAFT, 45764 Marl (DE)
(72) Erfinder: Brock, Michael, Dr., D-46514 Schermbeck (DE); Enneking, Meinolf, D-44627 Herne (DE)

(56) Entgegenhaltungen:
- EP-A- 0 331 085

## Beschreibung

Die Erfindung betrifft konzentrierte bei 40 °C fließfähige Polyethercarboxylate, ein Verfahren zu ihrer Herstellung und ihre Verwendung als Dispergier- und Emulgiermittel im Wasch- und Reinigungsmittelbereich.

Salze der Polyethercarbonsäuren sind nach van Paasen in Seifen Öle Fette Wachse 109 (1983), 353 besonders hautverträglich und werden daher zunehmend in Shampoos, Dusch- und Schaumbädern eingesetzt. Weiterhin zeichnen sich die Polyethercarboxylate durch eine außerordentliche Härtestabilität und sehr gute Dispergier- und Emulgiereigenschaften aus, so daß sie auch im Wasch- und Reinigungsmittelbereich und als technische Emulgatoren eingesetzt werden können.

Zur Herstellung von Polyethercarboxylaten sind im wesentlichen zwei Verfahren beschrieben. So lassen sie sich durch selektive Oxidation der endständig primären Hydroxylgruppe eines Oxethylats zu einer Carboxylgruppe unter basischen Bedingungen darstellen (DE-34 46 561). Verbreiteter ist jedoch die Synthese der Polyethercarboxylate durch Carboxymethylierung der Hydroxylgruppe des Oxethylats mit Natriumchloracetat in Gegenwart von Natriumhydroxid. Dabei werden die Natriumsalze der entsprechenden Polyethercarbonsäuren erhalten, die als Rohprodukte mit den Ausgangsprodukten sowie Natriumchlorid verunreinigt sind.

Das störende Natriumchlorid kann nach Kosswig und Stache, Die Tenside, Carl Hanser Verlag München · Wien, 1993, S. 123, nach Ansäuern z. B. mit Schwefelsäure abgetrennt werden. Das Natriumcarboxylat wird in die Polyethercarbonsäure überführt, die sich wegen ihrer Unlöslichkeit in Wasser über der sauren, wäßrigen Phase absetzen und praktisch frei von Chloridionen abtrennen läßt. Das Natriumchlorid verbleibt in der wäßrigen Phase. Die so erhaltenen Polyethercarbonsäuren sind wasserklare und leichtbewegliche Flüssigkeiten, die neben wenigen Gew.-% Restwasser und eventuell vorhandenem nicht umgesetzten Oxethylat keine weiteren nennenswerten Verunreinigungen enthalten.

Viele Verarbeiter sind jedoch nicht in der Lage, die aus transporttechnischer Sicht hervorragenden Polyethercarbonsäuren zu den Salzen zu neutralisieren, die allein Verwendung in den Fertigprodukten finden. Diese Verarbeiter sind daher nur an den Polyethercarboxylaten interessiert. Die üblicherweise verwendeten Natriumsalze der Polyethercarbonsäuren werden in der Regel bis zu Aktivgehalten von 25 Gew.-% angeboten. Höherkonzentrierte Produkte sind nicht mehr pumpbar und können somit nur schlecht verarbeitet werden. Produkte mit niedrigem Aktivgehalt verursachen hohe Distributionskosten, so daß es in der Vergangenheit nicht an Versuchen gefehlt hat, höherkonzentrierte und besser verarbeitbare Polyethercarboxylate zu entwickeln. So wird in DE-39 29 063 die Herstellung von entsprechenden Alkalisalzen mit Aktivgehalten bis zu 50 Gew.-% beschrieben. Diese Konzentrationserhöhung kann aber nicht als ausreichend betrachtet werden.

In der Patentschrift EP-0 344 442 wird die Herstellung von pumpbaren Produkten mit einem Aktivgehalt von über 70 Gew.-% beschrieben, denen aber kurzkettige Alkohole als Verdünner zugesetzt werden müssen. Deren Anwesenheit ist bei den Verarbeitern solcher Produkte in der Regel aber nicht erwünscht.

Aufgabe der vorliegenden Erfindung war es daher, ein Verfahren zur Herstellung von weitgehend Natriumchlorid-freien, fließfähigen und konzentrierten Polyethercarboxylaten zu entwickeln, denen kein Alkohol und auch keine anderen Additive zur Viskositätserniedrigung zugesetzt werden müssen.

Die Aufgabe wird erfindungsgemäß dadurch gelöst, daß konzentrierte Polyethercarbonsäuren mit entsprechenden alkalischen Verbindungen bei 40 bis 100 °C zu Polyethercarboxylaten neutralisiert werden und eine Temperierung des Polyethercarboxylatsalzes bei 40 bis 100 °C für bis zu 12 Stunden erfolgt. Dabei kann ein Vakuum von 1 bis 800 mbar, vorzugsweise 10 bis 600 mbar, angelegt werden und gegebenenfalls das Wasser wenigstens teilweise aus dem System entfernt werden.

Gegenstand der Erfindung sind mithin konzentrierte bei 40 °C fließfähige Polyethercarboxylate der Struktur I (Polyethercarboxylatkonzentrate)

R - O - (A - O)ₙ - CH₂ - COOM (I),

wobei
- R: einen Alkylrest mit 8 bis 24 Kohlenstoffatomen oder einen alkylsubstituierten Arylrest mit 9 bis 24 Kohlenstoffatomen und
- A: Alkylen mit 2 bis 5 Kohlenstoffatomen bedeuten,
- n: gleich 3 bis 10 ist und
- M: für ein Alkali-, ½ Erdalkali-, Ammonium- oder substituiertes Ammonium-Ion steht,
erhalten durch Neutralisation von konzentrierten Polyethercarbonsäuren der Struktur II

R - O - (A - O)ₙ - CH₂ - COOH (II),

wobei
- R: einen Alkylrest mit 8 bis 24 Kohlenstoffatomen oder einen alkylsubstituierten Arylrest mit 9 bis 24 Kohlenstoffatomen und
- A: Alkylen mit 2 bis 5 Kohlenstoffatomen bedeuten und
- n: gleich 3 bis 10 ist,
mit den entsprechenden alkalischen Verbindungen bei Temperaturen von 40 bis 100 °C und Temperierung des Polyethercarboxylatsalzes bei Temperaturen von 40 bis 100 °C für bis zu 12 Stunden, gegebenenfalls unter Anlegen eines Vakuums von 1 bis 800 mbar, vorzugsweise 10 bis 600 mbar, und Entfernen wenigstens eines Teils des verdampften Wassers.

Ein weiterer Gegenstand der Erfindung ist ein Verfahren zur Herstellung von konzentrierten bei 40 °C fließfähigen Polyethercarboxylatkonzentraten der Struktur I (Polyethercarboxylatkonzentrate)

R - O - (A - O)ₙ - CH₂ - COOM (I),

wobei
- R: einen Alkylrest mit 8 bis 24 Kohlenstoffatomen oder einen alkylsubstituierten Arylrest mit 9 bis 24 Kohlenstoffatomen und A Alkylen mit 2 bis 5 Kohlenstoffatomen bedeuten, n gleich 3 bis 10 ist und M für ein Alkali-, ½ Erdalkali-, Ammonium- oder substituiertes Ammonium-Ion steht,
dadurch gekennzeichnet,
daß man konzentrierte Polyethercarbonsäuren der Struktur II

R - O - (A - O)ₙ - CH₂ - COOH (II),

wobei R einen Alkylrest mit 8 bis 24 Kohlenstoffatomen oder einen alkylsubstituierten Arylrest mit 9 bis 24 Kohlenstoffatomen und A Alkylen mit 2 bis 5 Kohlenstoffatomen bedeuten und n gleich 3 bis 10 ist, mit den entsprechenden alkalischen Verbindungen bei Temperaturen von 40 bis 100 °C neutralisiert und bis zu 12 Stunden bei Temperaturen von 40 bis 100 °C, gegebenenfalls unter Anlegen eines Vakuums von 1 bis 800 mbar, vorzugsweise 10 bis 600 mbar, und Entfernen wenigstens eines Teils des verdampften Wassers, hält.

Die so erhaltenen Polyethercarboxylatkonzentrate haben einen Wasser-Gehalt von 0 bis 20 Gew.-%. Die Konzentration an Polyethercarboxylaten beträgt 50 bis 100 Gew.-%, vorzugsweise 65 bis 100 Gew.-%. Nicht zu Polyethercarboxylat umgesetztes Oxethylat kann bis zu 50 Gew.-% enthalten sein. Der Feststoffgehalt beträgt insgesamt bis zu 100 Gew.-%.

Überraschend ist die niedrige Viskosität und damit Pumpbarkeit der so hergestellten Polyethercarboxylatkonzentrate, die auf anderem Wege praktisch nicht herstellbar sind.

Unter Fließfähigkeit bei 40 °C im Sinne dieser Erfindung werden Lösungen oder Suspensionen verstanden, die bei 40 °C eine Viskosität von weniger als 50 000 mPa·s bei einem Schergefälle von 10 s⁻¹ ermittelt mit einem Haake-Rotationsviskosimeter, NV- bzw. SV-DIN, haben.

In den Polyethercarboxylaten der Struktur (I) ist R beispielsweise Decyl, Lauryl, Isotridecyl, Myristyl, Palmityl, Stearyl oder Nonylphenyl. Vorzugsweise steht R für Alkyl mit 10 bis 16 Kohlenstoffatomen. A ist vorzugsweise Ethylen und/oder Propylen. In einer besonders bevorzugten Ausführungsform steht A für Ethylen.

Beispiele für M sind Kalium-, Natrium-, Ammonium-, Magnesium- oder substituierte Ammonium-Ionen wie Trimethylammonium, Triethanolammonium, Mono-, Di- und Triisopropanolammonium. Vorzugsweise steht M für das Natrium-Ion. Die Neutralisation wird daher mit den entsprechenden alkalischen Verbindungen, wie z. B. Natronlauge, Kalilauge, Magnesiumoxid, Magnesiumhydroxid oder Trimethylamin durchgeführt.

Die erfindungsgemäßen Polyethercarboxylatkonzentrate haben einen hohen Gehalt an Aktivsubstanz, sind praktisch frei von Natriumchlorid und bei 40 °C fließfähig. Mit "praktisch frei von Natriumchlorid" wird ein Gehalt des Natriumchlorids von höchstens 0,5 Gew.-% bezeichnet.

Die folgenden Beispiele sollen die Erfindung verdeutlichen, ohne diese einzuschränken.

### Beispiel 1:

In einem 500 ml Rundkolben, ausgestattet mit Thermometer, Tropftrichter und Claisen-Brücke, wurden 200 g eines Gemisches aus 86,3 Gew.-% C₁₂-/C₁₄-Alkyl-4 mol EO-Ethercarbonsäure, 6 Gew.-% C₁₂-/C₁₄-Alkyl-4 mol EO-Addukt, 0,1 Gew.-% NaCl und 7,6 Gew.-% Wasser bei 80 °C und einem Vakuum von 440 mbar langsam mit 32,2 g einer wäßrigen 50%igen NaOH-Lösung versetzt. Nach beendeter Zugabe der NaOH-Lösung wurde 4 Stunden bei 50 mbar und 80 °C nachgerührt.

Die Analyse des erhaltenen Produktes zeigt die folgende Zusammensetzung an:

| | |
|---|---|
| Trockengehalt: | 99,7 Gew.-% |
| Polyethercarboxylat-Na-salz: | 93,4 Gew.-% |
| Neutralölgehalt: | 5,8 Gew.-% |
| NaCl-Gehalt: | 0,2 Gew.-% |
| Wasser-Gehalt: | 0,3 Gew.-% |

Das Produkt wurde in mehrere Proben aufgeteilt, die mit verschiedenen Mengen Wasser versetzt wurden. Von den Proben wurden die Viskositäten bei 40 °C und einen Schergefälle von 10 s⁻¹ ermittelt (Haake-Rotationsviskosimeter, NV- bzw. SV-DIN). Die gefundenen Viskositäten sind nachfolgend tabellarisch aufgeführt:

| | **Viskosität [mPa·s]** |
|---|---|
| Probe 1 (Wassergehalt 0,3 Gew.-%) | 4 000 |
| Probe 2 (Wassergehalt 2 Gew.-%) | 2 500 |
| Probe 3 (Wassergehalt 4 Gew.-%) | 1 500 |
| Probe 4 (Wassergehalt 6 Gew.-%) | 1 000 |
| Probe 5 (Wassergehalt 8 Gew.-%) | 1 000 |
| Probe 6 (Wassergehalt 10 Gew.-%) | 2 500 |
| Probe 7 (Wassergehalt 12 Gew.-%) | 5 500 |
| Probe 8 (Wassergehalt 16 Gew.-%) | 10 000 |
| Probe 9 (Wassergehalt 22 Gew.-%) | > 100 000 |

Die Viskositäten der Proben 1 bis 8 sind bei 40 °C fließfähig, die nicht erfindungsgemäße Probe 9 nicht.

### Beispiel 2:

Analog den in Beispiel 1 genannten Versuchsbedingungen wurden 200 g eines Gemisches aus 62,8 Gew.-% C₁₂-/C₁₄-Alkyl-4,5 mol EO-Ethercarbonsäure, 26,1 Gew.-% C₁₂-/C₁₄-Alkyl-4,5 mol EO-Addukt, 0,1 Gew.-% NaCl und 11 Gew.-% Wasser mit 22,4 g einer wäßrigen 50%igen NaOH-Lösung versetzt. Die anschließend durchgeführte Analyse ergab folgende Produktzusammensetzung:

| | |
|---|---|
| Trockengehalt: | 98,8 Gew.-% |
| Polyethercarboxylat-Na-salz: | 72,1 Gew.-% |
| Neutralölgehalt: | 26,3 Gew.-% |
| NaCl-Gehalt: | 0,2 Gew.-% |
| Wasser-Gehalt: | 1,2 Gew.-% |

Das erhaltene Produkt wurde, wie bereits in Beispiel 1 erwähnt, geteilt und mit verschiedenen Mengen Wasser versetzt. Die anschließend durchgeführten Viskositätsmessungen ergaben folgende Werte (Meßbedingungen s. Beispiel 1):

| | **Viskosität [mPa·s]** |
|---|---|
| Probe 1 (Wassergehalt 1,2 Gew.-%) | 300 |
| Probe 2 (Wassergehalt 4 Gew.-% ) | 300 |
| Probe 3 (Wassergehalt 6 Gew.-%) | 300 |
| Probe 4 (Wassergehalt 8 Gew.-%) | 300 |
| Probe 5 (Wassergehalt 10 Gew.-%) | 300 |
| Probe 6 (Wassergehalt 12 Gew.-%) | 300 |
| Probe 7 (Wassergehalt 15 Gew.-%) | 2 500 |
| Probe 8 (Wassergehalt 22 Gew.-%) | 50 000 |

Die Proben 1 bis 7 sind bei 40 °C fließfähig, die nicht erfindungsgemäße Probe 8 nicht.

## Patentansprüche

1. Konzentrierte bei 40 °C fließfähige Polyethercarboxylate der Struktur I (Polyethercarboxylatkonzentrate)
R - O - (A - O)ₙ - CH₂ - COOM (I),
wobei
R einen Alkylrest mit 8 bis 24 Kohlenstoffatomen oder einen alkylsubstituierten Arylrest mit 9 bis 24 Kohlenstoffatomen und
A Alkylen mit 2 bis 5 Kohlenstoffatomen bedeuten,
n gleich 3 bis 10 ist und
M für ein Alkali-, ½ Erdalkali-, Ammonium- oder substituiertes Ammonium-Ion steht,
erhalten durch Neutralisation von konzentrierten Polyethercarbonsäuren der Struktur II
R - O - (A - O)ₙ - CH₂ - COOH (II),
wobei
R einen Alkylrest mit 8 bis 24 Kohlenstoffatomen oder einen alkylsubstituierten Arylrest mit 9 bis 24 Kohlenstoffatomen und
A Alkylen mit 2 bis 5 Kohlenstoffatomen bedeuten und
n gleich 3 bis 10 ist,
mit den entsprechenden alkalischen Verbindungen bei Temperaturen von 40 bis 100 °C und Temperierung des Polyethercarboxylatsalzes bis zu 12 Stunden bei Temperaturen von 40 bis 100 °C.

2. Polyethercarboxylatkonzentrate nach Anspruch 1,
dadurch gekennzeichnet,
daß bei der Neutralisation bei Temperaturen von 40 bis 100 °C ein Vakuum von 1 bis 800 mbar, vorzugsweise 10 bis 600 mbar, angelegt wird.

3. Polyethercarboxylatkonzentrate nach Anspruch 1,
dadurch gekennzeichnet,
daß nach der Neutralisation während der Temperierung bei Temperaturen von 40 bis 100 °C ein Vakuum von 1 bis 800 mbar, vorzugsweise 10 bis 600 mbar, angelegt wird.

4. Polyethercarboxylatkonzentrate nach mindestens einem der Ansprüche 1 bis 3,
dadurch gekennzeichnet,
daß bei der Neutralisation und während der Temperierung verdampftes Wasser entfernt wird.

5. Polyethercarboxylatkonzentrate nach mindestens einem der Ansprüche 1 bis 4,
dadurch gekennzeichnet,
daß die Konzentration an Polyethercarboxylaten 65 bis 100 Gew.-% beträgt.

6. Polyethercarboxylatkonzentrate nach mindestens einem der Ansprüche 1 bis 4,
dadurch gekennzeichnet,
daß der Wassergehalt der Konzentrate 0 bis 20 Gew.-% beträgt.

7. Polyethercarboxylatkonzentrate nach mindestens einem der Ansprüche 1 bis 4,
dadurch gekennzeichnet,
daß der Gehalt des Polyethercarboxylats an nicht umgesetzten Oxethylat 0 bis 50 Gew.-% beträgt.

8. Polyethercarboxylatkonzentrate nach mindestens eines der Ansprüche 1 bis 4,
dadurch gekennzeichnet,
daß R in den Formeln I und II einen Alkylrest mit 10 bis 16 Kohlenstoffatomen bedeutet.

9. Polyethercarboxylatkonzentrate nach mindestens einem der Ansprüche 1 bis 4,
dadurch gekennzeichnet,
daß A in den Formeln I und II Ethylen und/oder Propylen bedeutet.

10. Polyethercarboxylatkonzentrate nach mindestens einem der Ansprüche 1 bis 4,
dadurch gekennzeichnet,
daß M in der Formel I ein Alkali- oder Ammonium-Ion ist.

11. Verfahren zur Herstellung von konzentrierten bei 40 °C fließfähigen Polyethercarboxylaten der Struktur I (Polyethercarboxylatkonzentrate)
R - O - (A - O)ₙ - CH₂ - COOM (I),
wobei
R einen Alkylrest mit 8 bis 24 Kohlenstoffatomen oder einen alkylsubstituierten Arylrest mit 9 bis 24 Kohlenstoffatomen und A Alkylen mit 2 bis 5 Kohlenstoffatomen bedeuten, n gleich 3 bis 10 ist und M für ein Alkali-, ½ Erdalkali-, Ammonium- oder substituiertes Ammonium-Ion steht,
dadurch gekennzeichnet,
daß man konzentrierte Polyethercarbonsäuren der Struktur II
R - O - (A - O)ₙ - CH₂ - COOH (II),
wobei R einen Alkylrest mit 8 bis 24 Kohlenstoffatomen oder einen alkylsubstituierten Arylrest mit 9 bis 24 Kohlenstoffatomen und A Alkylen mit 2 bis 5 Kohlenstoffatomen bedeuten und n gleich 3 bis 10 ist, mit den entsprechenden alkalischen Verbindungen bei Temperaturen von 40 bis 100 °C neutralisiert und bis zu 12 Stunden bei Temperaturen von 40 bis 100 °C hält.

12. Verfahren zur Herstellung von Polyethercarboxylatkonzentraten nach Anspruch 11,
dadurch gekennzeichnet,
daß man bei der Neutralisation und/oder bis zu 12 Stunden danach bei Temperaturen von 40 bis 100 °C ein Vakuum von 1 bis 800 mbar, vorzugsweise 10 bis 600 mbar, anlegt.

13. Verfahren zur Herstellung von Polyethercarboxylatkonzentraten nach mindestens eines der Ansprüche 11 und 12,
dadurch gekennzeichnet,
daß man bei der Neutralisation und während der Temperierung bei Temperaturen von 40 bis 100 °C verdampftes Wasser wenigstens teilweise entfernt.

14. Verwendung der Polyethercarboxylate, hergestellt nach mindestens eines der Ansprüche 11 bis 13 als Dispergier- und Emulgiermittel im Wasch- und Reinigungsmittelbereich.

## Claims

1. A concentrated polyethercarboxylate flowable at 40°C of the structure I (polyethercarboxylate concentrate)
R - O - (A - O)ₙ - CH₂ - COOM (I),
in which
R denotes an alkyl radical having 8 to 24 carbon atoms or an alkyl-substituted aryl radical having 9 to 24 carbon atoms and
A denotes alkylene having 2 to 5 carbon atoms,
n is equal to 3 to 10 and
M represents an alkali metal ion, ½ alkaline earth metal ion, ammonium ion or substituted ammonium ion,
obtained by neutralization of concentrated polyethercarboxylic acids of the structure II
R - O - (A - O)ₙ - CH₂ - COOH (II),
in which
R denotes an alkyl radical having 8 to 24 carbon atoms or an alkyl-substituted aryl radical having 9 to 24 carbon atoms and
A denotes alkylene having 2 to 5 carbon atoms and
n is equal to 3 to 10,
with the corresponding alkaline compounds at temperatures of 40 to 100°C and maintaining the polyethercarboxylate salt for up to 12 hours at temperatures of 40 to 100°C.

2. A polyethercarboxylate concentrate according to claim 1, characterized in that in the neutralization at temperatures of 40 to 100°C a vacuum of 1 to 800 mbar, preferably 10 to 600 mbar, is applied.

3. A polyethercarboxylate concentrate according to claim 1, characterized in that after the neutralization, while the product is being maintained at temperatures of 40 to 100°C, a vacuum of 1 to 800 mbar, preferably 10 to 600 mbar, is applied.

4. A polyethercarboxylate concentrate according to at least one of claims 1 to 3, characterized in that in the neutralization and during the maintenance at specified temperatures evaporated water is removed.

5. A polyethercarboxylate concentrate according to at least one of claims 1 to 4, characterized in that the concentration of polyethercarboxylate is 65 to 100% by weight.

6. A polyethercarboxylate concentrate according to at least one of claims 1 to 4, characterized in that the water content of the concentrate is 0 to 20% by weight.

7. A polyethercarboxylate concentrate according to at least one of claims 1 to 4, characterized in that the content of unreacted ethoxylate in the polyethercarboxylate is 0 to 50% by weight.

8. A polyethercarboxylate concentrate according to at least one of claims 1 to 4, characterized in that R in the formulae I and II denotes an alkyl radical having 10 to 16 carbon atoms.

9. A polyethercarboxylate concentrate according to at least one of claims 1 to 4, characterized in that A in the formulae I and II denotes ethylene and/or propylene.

10. A polyethercarboxylate concentrate according to at least one of claims 1 to 4, characterized in that M in the formula I is an alkali metal ion or ammonium ion.

11. A process for the preparation of concentrated polyethercarboxylates flowable at 40°C of the structure I (polyethercarboxylate concentrates)
R - O - (A - O)ₙ - CH₂ - COOM (I),
in which
R denotes an alkyl radical having 8 to 24 carbon atoms or an alkyl-substituted aryl radical having 9 to 24 carbon atoms and A denotes alkylene having 2 to 5 carbon atoms, n is equal to 3 to 10 and M represents an alkali metal ion, ½ alkaline earth metal ion, ammonium ion or substituted ammonium ion,
characterized in that
concentrated polyethercarboxylic acids of the structure II
R - O - (A - O)ₙ - CH₂ - COOH (II),
in which
R denotes an alkyl radical having 8 to 24 carbon atoms or an alkyl-substituted aryl radical having 9 to 24 carbon atoms and A denotes alkylene having 2 to 5 carbon atoms and n is equal to 3 to 10, are neutralized with the corresponding alkaline compounds at temperatures of 40 to 100°C and kept for up to 12 hours at temperatures of 40 to 100°C.

12. A process for the preparation of polyethercarboxylate concentrates according to claim 11, characterized in that in the neutralization and/or up to 12 hours afterwards at temperatures of 40 to 100°C a vacuum of 1 to 800 mbar, preferably 10 to 600 mbar, is applied.

13. A process for the preparation of polyethercarboxylate concentrates according to at least one of claims 11 and 12, characterized in that in the neutralization and during the maintenance at temperatures of 40 to 100°C, evaporated water is at least partially removed.

14. The use of the polyethercarboxylates prepared according to at least one of claims 11 to 13 as dispersants and emulsifiers in the detergent and cleaning composition sector.

## Revendications

1. Polyéthercarboxylates concentrés aptes à l'écoulement à 40°C, de structure I (concentrés de polyéthercarboxylate)
R - O (A - O)ₙ - CH₂ - COOM (I)
dans laquelle,
R signifie un radical alkyle ayant de 8 à 24 atomes de carbone ou un radical aryle substitué par un alkyle, ayant de 9 à 24 atomes de carbone,
A signifie un alkylène ayant de 2 à 5 atomes de carbone,
n est égal de 3 à 10 et,
M représente un ion de métal alcalin, un ½ ion de métal alcalino-terreux, un ion ammonium, ou un ion ammonium substitué, obtenus par neutralisation d'acides polyéthercarboxyliques concentrés de structure II,
R - O (A - O)ₙ - CH₂ - COOH (II)
dans laquelle,
R signifie un radial alkyle ayant de 8 à 24 atomes de carbone ou un radical aryle substitué par un alkyle ayant de 9 à 24 atomes de carbone et,
A signifie un alkylène ayant de 2 à 5 atomes de carbone et, n est égal de 3 à 10
avec les composés alcalins correspondants à des températures allant de 40 à 100°C et en équilibre de température du sel de polyéthercarboxylate jusqu'à 12 heures à des températures s'échelonnant de 40 à 100°C.

2. Concentrés de polyéthercarboxylate selon la revendication 1,
caractérisés en ce qu'
on applique au cours de la neutralisation à des températures allant de 40 à 100°C, un vide de 1 à 800 mbars, de préférence de 10 à 600 mbars.

3. Concentrés de polyéthercarboxylates selon la revendication 1,
caractérisés en ce qu'
on applique après la neutralisation pendant l'équilibrage de température à des températures de 40 à 100°C, un vide de 1 à 800 mbars, de préférence de 10 à 600 mbars.

4. Concentrés de polyéthercarboxylates selon au moins une des revendications 1 à 3,
caractérisés en ce qu'
au cours de la neutralisation et pendant l'équilibrage de température, on élimine l'eau évaporée.

5. Concentrés de polyéthercarboxylates selon au moins une des revendications 1 à 4,
caractérisés en ce que
la concentration en polyéthercarboxylates s'élève de 65 à 100 % en poids.

6. Concentrés de polyéthercarboxylates selon au moins une des revendications 1 à 4,
caractérisés en ce que
la teneur en eau des concentrés s'élève de 0 à 20 % en poids.

7. Concentrés de polyéthercarboxylates selon au moins une des revendications 1 à 4
caractérisés en ce que
la teneur du polyéthercarboxylate en oxoéthylate qui n'a pas réagi s'élève de 0 à 50 % en poids.

8. Concentrés de polyéthercarboxylates selon au moins une des revendications 1 à 4,
caractérisés en ce que
R dans les formules I et II signifie un radical alkyle ayant de 10 à 16 atomes de carbone.

9. Concentrés de polyéthercarboxylates selon au moins une des revendications 1 à 4,
caractérisés en ce que
A dans les formules I et II signifie l'éthylène et/ou le propylène.

10. Concentrés de polyéthercarboxylates selon au moins une des revendications 1 à 4,
caractérisés en ce que
M dans la formule I est un ion de métal alcalin ou d'ammonium.

11. Procédé de fabrication de polyéthercarboxylates concentrés, aptes à l'écoulement à 40°C, de structure I (concentré de polyéthercarboxylate).
R - O (A - O)ₙ - CH₂ - COOM (I)
dans laquelle R signifie un radical alkyle ayant de 8 à 24 atomes de carbone ou un radical aryle substitué par un alkyle, ayant de 9 à 24 atomes de carbone,
et A signifie un alkylène ayant de 2 à 5 atomes de carbone, n est égal de 3 à 10 et,
M représente un ion de métal alcalin, ½ d'ion alcalino-terreux, un ion ammonium ou un ion ammonium substitué, caractérisé en ce qu'
on concentre des acides polyéthercarboxyliques concentrés de structure II,
R - O (A - O)ₙ - CH₂ - COOH (II)
dans laquelle R signifie un radical alkyle ayant de 8 à 24 atomes de carbone ou un radical aryle substitué par un alkyle ayant de 9 à 24 atomes de carbone et,
A signifie un alkylène ayant de 2 à 5 atomes de carbone et, n est égale de 3 à 10,
neutralisé par les composés alcalins correspondants à des températures allant de 40 à 100°C et on maintient jusqu'à 12 heures à des températures allant de 40 à 100°C.

12. Procédé de fabrication de concentrés de polyéthercarboxylates selon la revendication 11,
caractérisé en ce qu'
on applique au cours de la neutralisation et/ou jusqu'à 12 heures ensuite, à des températures allant de 40 à 100°C, un vide de 1 à 800 mbars, de préférence de 10 à 600 mbars.

13. Procédé de fabrication de concentrés de polyéthercarboxylate selon au moins une des revendications 11 et 12,
caractérisé en ce qu'
on élimine au cours de la neutralisation et pendant l'équilibrage de température, à des températures allant de 40 à 100°C, l'eau évaporée, au moins partiellement.

14. Utilisation des polyéthercarboxylates produits selon au moins une des revendications 11 à 13, comme agents dispersants et émulsionnants, dans le domaine des produits de lavage et de nettoyage.
